# EUROPEAN PATENT APPLICATION

(11) **EP 4 623 890 A1**
(43) Date of publication of application: **01.10.2025**
(21) Application number: 25161638.9
(22) Date of filing: 04.03.2025
(51) Int. Cl.: A61K 8/02, A61C 3/025, A61K 8/19, A61Q 11/00

(54) **POWDER AND LIQUID COMPOSITIONS FOR DENTAL CARE, AND METHODS OF USE THEREOF**

(30) Priority: 05.03.2024 US 202463561612 P
(71) Applicant: More Group Pty. Ltd., Melbourne, VIC 3004 (AU)
(72) Inventor: NADGORNY, Milena, Melbourne, 3142 (AU); GILMORE, Ruth, Melbourne, 3142 (AU)
(74) Representative: Novitas Patent AB

(57) **Abstract**

Some aspects described herein provide power and liquid compositions, which may be utilized for dental care. Some aspects provide small grained compositions that include activated carbon with small particle size, which may be used for dental care. One aspect includes a dental cleaning powder. For example, the dental cleaning powder includes activated carbon. For example, the activated carbon may have a D₉₀ particle size of between approximately 25 µm and approximately 45µm. Another aspect includes a method for air polishing, which includes injecting to an oral cavity an aqueous solution of one or more water-soluble enzymes via a liquid compartment of an air polishing device.

## Description

### CROSS REFERENCE

This Application claims the benefit of and priority from US Provisional Patent Application No. 63/561,612 entitled "POWDER AND LIQUID COMPOSITION OF DENTAL CARE FORMULATIONS", filed March 5, 2024, the entire disclosure of which is incorporated herein by reference.

### BACKGROUND

Biofilm management is one of the key challenges in maintaining a healthy periodontium. Powder jet cleaning is a method used for supra and sub-gingival cleaning of hard dental tissue from biofilm plaque, stains and deposits. The method is becoming broadly adopted by dental professionals owing to it causing less discomfort to patients than traditional methods of mechanical scraping or ultrasonic cleaning.

### BRIEF DESCRIPTION OF THE DRAWINGS

For simplicity and clarity of illustration, elements shown in the figures have not necessarily been drawn to scale. For example, the dimensions of some of the elements may be exaggerated relative to other elements for clarity of presentation. Furthermore, reference numerals may be repeated among the figures to indicate corresponding or analogous elements. The figures are listed below.
Fig. 1 displays a differential volume graph showing the distribution of particle size of a powder composition, in accordance with some demonstrative aspects.
Fig. 2 shows a Scanning Electron Microscopy (SEM) micrograph of a powder, in accordance with some demonstrative aspects.
Fig 3 shows human molar teeth before and after air polishing, in accordance with some demonstrative aspects.
Fig 4. shows post air polishing treatment dentine wear of a human molar, in accordance with some demonstrative aspects.
Fig. 5 schematically illustrates an air polishing device that can be used in a method, in accordance with some demonstrative aspects.

### DETAILED DESCRIPTION

Formulations used for powder jet dental cleaning (also referred to as air polishing) may include abrasive powders based on sodium bicarbonate, glycine, polyols, such as erythritol, polysaccharides, or bioactive glass. The particles of these powders typically exhibit an abrasive nature, and can cause damage to enamel, dentin and cementum, limiting their applicability to supra-gingival cleaning only. Furthermore, sodium bicarbonate is corrosive to several restoration materials, such as amalgam, and is contraindicated to patients diagnosed with hypertension.

Mildly abrasive powders may be used to address the need for sub-gingival cleaning of periodontal pockets, and may have a high significance for treatment of patients with gum recession, peri-implantitis, and other periodontal diseases. Such powders, include, for example, glycine, erythritol and polysaccharides.

Unfortunately, the arsenal of known cleaning materials in powdered form is still limited, mainly because of technical challenges related to particle size, flow properties and abrasive potential. Therefore, some promising cleaning candidates cannot be introduced to the powder compartment of air polishing equipment.

The awareness to the importance of dental prophylaxis is increasing, therefore, there is an increasing demand for effective cleaning formulas that can be applied on dental surfaces, including periodontal pockets and restorative materials, minimizing the abrasive damage and patients' discomfort.

Some demonstrative aspects provide small grained compositions that include activated carbon with small particle size, for dental care, e.g., biofilm removal and dental prophylaxis. The compositions, according to some aspects, meet abrasion safety criteria for the teeth, are safe for application on dentine with minimal abrasion and low surface damage, and can be easily and safely used with air polishing devices, e.g., as described below.

In some demonstrative aspects, a dual-action cleaning may be achieved by using a liquid composition of enzymes in the liquid compartment of an air polishing device, thereby improving the cleaning potential, e.g., as described below.

In some demonstrative aspects, a dental cleaning powder may include activated carbon having a D₉₀ particle size of between approximately 25 µm and approximately 45µm.

In some demonstrative aspects, the activated carbon typically has a mean particle size of 10-100 µm, e.g., as described below.

In some demonstrative aspects, the activated carbon has a mean particle size of 10-30 µm, e.g., as described below.

In some demonstrative aspects, the activated carbon includes steam processed coconut shell, e.g., as described below.

In some demonstrative aspects, the dental cleaning powder includes about 0.1% - 5% weight of the activated carbon, e.g., as described below.

In some demonstrative aspects, the dental cleaning powder includes 0.1% - 0.5% weight of the activated carbon, e.g., as described below.

In some demonstrative aspects, the dental cleaning powder may include additional agents such as an anticaking agent (having a mean particle size of 1-100 µm) in an amount of up to approximately 10% weight and/or a cleaning agent, which may include one or more of: a sugar alcohol, an amino acid, a sugar and an alkaline salt, in an amount of between 82% to 99.5% w/w, e.g., as described below.

In some demonstrative aspects, the cleaning agent has a mean particle size of 5-500 µm. An additional agent, including, e.g., an antimicrobial agent, may be present in an amount of up to approximately 3% weight, e.g., as described below.

In one example, a dental cleaning powder includes 99 - 99.4% w/w erythritol, 0.1 - 0.5% w/w activated carbon and 0.5% w/w silica, e.g., as described below.

In some demonstrative aspects, the dental cleaning powder has a D₅₀ particle size of between approximately 20 µm and approximately 80 µm. In one aspect, the dental cleaning powder has a D₉₀ particle size of approximately 54.5 µm, e.g., as described below.

Some demonstrative aspects include the use of a dental cleaning powder, which includes activated carbon, in airflow-assisted dental care.

In some demonstrative aspects, the dental care may be subgingival, in which case the powder has a D₅₀ particle size of approximately 20 µm and/or the dental care may be supra-gingival, in which case the powder has a D₅₀ particle size of approximately 80 µm, e.g., as described below.

Some demonstrative aspects include an air polishing powder which includes activated carbon particles, e.g., as described below.

In some demonstrative aspects, the activated carbon particles may typically have a mean particle size of 10-30 µm and a D₉₀ of approximately 45 µm, e.g., as described below.

Some demonstrative aspects include a method for air polishing, e.g., as described below.

In some demonstrative aspects, the method includes injecting to an oral cavity an aqueous solution of one or more water-soluble enzymes via an air polishing device, e.g., as described below.

In some demonstrative aspects, the water-soluble enzymes may include one or more of: amylase, cellulase, lysozyme, papain, bromelain and ficin, e.g., as described below.

In some demonstrative aspects, the aqueous solution may typically include up to 5% w/v of the one or more water-soluble enzyme, e.g., as described below.

In one example, the aqueous solution comprises about 0.4 -0.5% w/v of the one or more water-soluble enzyme, e.g., as described below.

In one example, the aqueous solution includes 0.4% w/v bromelain and 99.6% water, e.g., as described below.

In some demonstrative aspects, the method may include expelling to the oral cavity, via the air polishing device, a powder which includes activated carbon particles, e.g., as described below.

In some demonstrative aspects, the aqueous solution and the powder may be expelled into the oral cavity substantially simultaneously, e.g., as described below.

Compositions and use of the compositions, according to some aspects, may be implemented to provide improved dental cleaning and oral prophylaxis. Cleaning compositions, according to some aspects, are typically small grained powders that include activated carbon with small particle size, which meet abrasion safety criteria for the teeth, are safe for application on dentine, and can be safely used with air polishing devices. In addition, a dual-action cleaning may be achieved by using a liquid composition of enzymes, e.g., in the liquid compartment of an air polishing device, to be applied to the oral cavity, possibly, in concert with a cleaning powder, according to some aspects.

In one aspect, a dental cleaning powder includes activated carbon. Activated carbon has a high surface area and porosity and can, therefore, efficiently absorb chemicals, impurities and toxins, making it beneficial for dental cleaning, e.g., in air polishing processes. For example, activated carbon can effectively absorb toxic components which are present in biofilm matrix as well as efficiently adsorb bacteria and yeast. Thus, some aspects may be implemented to provide a solution for, inter alia, an issue of bacteria growing in biofilm, which may exhibit increased resistance to antimicrobial agents.

The abrasive potential of activated carbon in cleaning powder compositions according to some aspects, is controlled by its particle size distribution. In some aspects, an ultra-fine powder of activated carbon may be used to enable safe application.

The term activated carbon typically includes a group of materials which are produced by pyrolysis of carbon-containing material, followed by activation. Activation may include, for example, chemical activation, steam processing, or the like.

In some demonstrative aspects, a dental cleaning powder includes activated carbon with particles having a D₉₀ size of between approximately 25 µm and approximately 45µm.

In some demonstrative aspects, the activated carbon has a mean particle size of 10-100 µm.

In one aspect, the activated carbon has a mean particle size of 10-30 µm. A non-limiting example of activated carbon used in some aspects, includes steam processed coconut shell activated carbon (e.g., as provided by Haycarb Holdings Australia Pty Ltd). In other aspects, any other suitable activated carbon may be used.

In some demonstrative aspects, a dental cleaning powder may include 0.1% - 5% weight of activated carbon.

In one aspect, the dental cleaning powder includes 0.1% weight of activated carbon.

In another aspect, the dental cleaning powder includes 0.5% weight of activated carbon.

In some demonstrative aspects, the dental cleaning powder may include an amount of activated carbon within the range of 0.1%- 0.5% w/w.

In some demonstrative aspects, a dental cleaning powder may have a D₅₀ of between approximately 20 µm and approximately 80 µm. For example, the dental cleaning powder may have a D₅₀ of 20 µm, 50 µm, 80 µm, or any other value within this range. In one aspect, the dental cleaning powder has a D₉₀ of approximately 54.5 µm.

In some demonstrative aspects, a dental cleaning powder may include, in addition to activated carbon, one or more (e.g., some or all) of the following ingredients:
1) A cleaning agent which may contribute to the cleaning properties of the powder. The cleaning agent may include, for example, one or more of: sugar alcohols, amino acids, sugars (disaccharides or polysaccharides), and alkaline salts. Some examples of sugar alcohols that may be used in some aspects, include erythritol, xylitol, sorbitol, mannitol, and threitol. Polysaccharides may include, for example, glucose, fructose, saccharose, trehalose and palatinose. Amino acids that may be used, in some aspects, include, for example, glycine, alanine, glutamine, glutamic acid, asparagine, aspartic acid, serine, valine, and leucine. Examples of alkaline salts may include sodium bicarbonate and potassium bicarbonate. In some aspects, the cleaning agent typically includes particles in the size range of 5 to 500 µm. The cleaning component may comprise 82% to 99.5% w/w of the dental cleaning powder, according to some aspects. In one example, the dental cleaning powder includes 90-98% w/w erythritol with a mean particle size of 10-60 µm.
2) An anti-caking agent, which may impart improved flow properties to a powder and prevent particles agglomeration. The anti-caking agent may include, for example, a solid additive that facilitates a free flow of powders, e.g., by disrupting the interactions between particles, preventing the formation of agglomerates and/or lumps. Some examples of anti-caking agents which may be used in some aspects, include fumed silica, chemically prepared silica and hollow or solid glass beads. In some aspects, the mean particle size of anti-caking agents is typically in the range of 1-100 µm. The anti-caking agents may comprise 0.05-10% w/w of the dental cleaning powder. In one example, the dental cleaning powder includes 0.1-0.5% w/w silica with a mean particle size of 1-30 µm.
3) Additives, such as one or more antimicrobial agent, one or more colorant, one or more flavoring agent, one or more homeostatic agent, one or more gelling agent, one or more pharmaceutical agent, etc. For example, the addition of anti-microbial agents to the dental cleaning powder may be beneficial to reduce the risk of infection. Examples of antimicrobial agents which may be used in some aspects, may include: Cetypyridiniumcloride (CPC), Chlorhexidin (CHX), Triclosan, alcohols, organic acids, quaternary ammonium compounds and/or silver containing compounds. Examples of homeostatic agents may include oxides, chloride or sulphate salts, polyphenols and zinc. A possible colorant may include, for example, FD&C Blue no. 1 (triarylmethane dye). Examples of gelling agents may include polymers, proteins, gums, fatty acids esters, clays, or combinations thereof. Dental cleaning powders, according to some aspects, may include between 0 to 3% w/w of an additive, e.g., as described above.

As described herein, cleaning powders according to some aspects are typically small grained and possess flow properties that enable them to be safely and efficiently used for dental cleaning and oral prophylaxis in air polishing device-assisted dental care, namely, in an air polishing process. Thus, some aspects provide an air polishing powder which includes activated carbon particles.

In some demonstrative aspects, air polishing powders include 0.1-5% w/w activated carbon, 82-99.5 w/w cleaning agent (e.g., ingredient 1 as described herein), 0.05% - 10% w/w anti-caking agent (e.g., ingredient 2 as described herein) and 0-3% w/w additive (e.g., ingredient 3 as described herein).

Powders in accordance with some aspects may be used in subgingival and/or supra-gingival dental care.

In some demonstrative aspects, for subgingival dental care, a cleaning powder has a D₅₀ of approximately 20 µm. For supra-gingival dental care, the powder may have a D₅₀ of approximately 80 µm. However, some variations of these values are acceptable. In other cases, other suitable values may be used.

Some demonstrative aspects are further illustrated by the following examples and figures, which are provided to facilitate understanding but are not intended to limit the scope of the aspects described herein.

### EXAMPLES

### Example 1 - composition of Powder 1

**Table 1**

| Component | % (w/w) |
|---|---|
| Component A: Erythritol (CAS # 149-32-6) | 99 |
| Component B: Activated carbon (CAS# 7440-44-0) | 0.5 |
| Component C: Silica (CAS # 112945-52-5) | 0.5 |

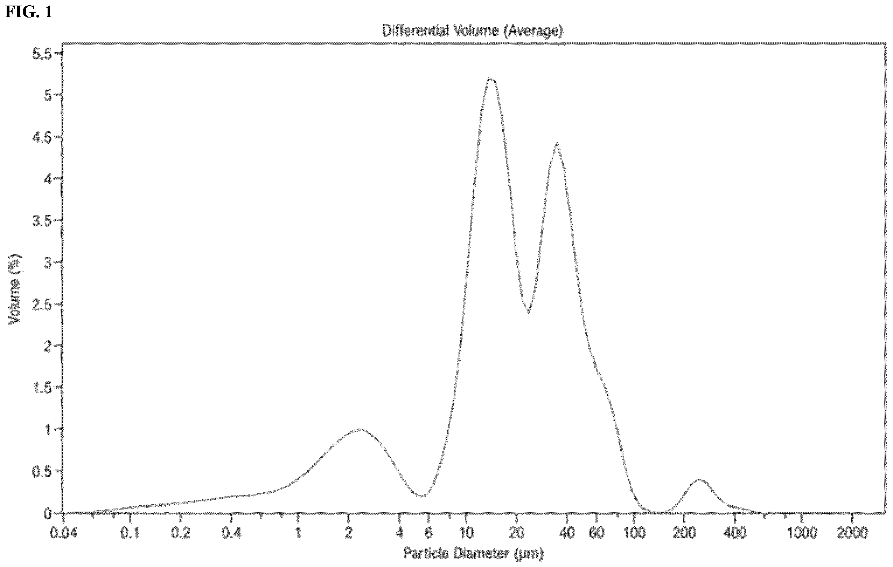

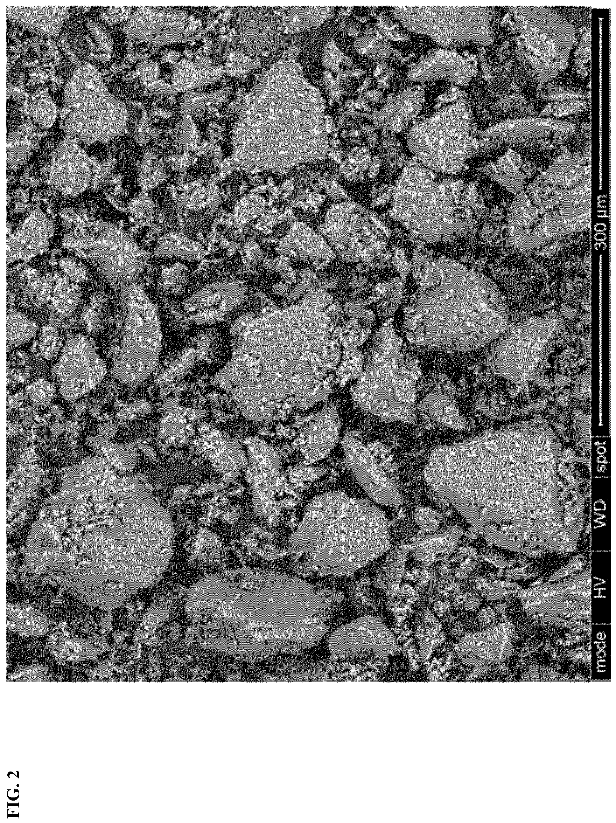

As demonstrated in Fig. 1 and Fig. 2, the mean particle size of Powder 1 is 28.8 µm, with a median particle size of 17.4 µm. Namely, 50% of the particles (D₅₀) in the powder are smaller than 17.4µm. 90% of the particles (D₉₀) in the powder are smaller than 54.5µm.

Fig. 1 depicts a differential volume graph showing the distribution of particle size in Powder 1 as analyzed by using a laser light scattering method according to ISO 13320 with a Coulter^{™} LS230 instrument. The calculations were carried out for particle sizes of 0.0400µm to 2,000µm, with 100% of the total particle volume falling within this range. The mean particle size was determined to be 28.83µm (S.D. 42.34 µm), the median 17.41µm (C.V. 147%) and the mode 13.61µm. D(3,2) (Sauter Mean Diameter (SMD)) was 3.843µm. 10% of the particles (D₁₀) were determined to be smaller than 2.077µm. 25% of the particles (D₂₅) were determined to be smaller than 10.61µm. 50% of the particles (D₅₀) were determined to be smaller than 17.41µm. 75% of the particles (D₇₅) were determined to be smaller than 35.27µm and 90% of the particles (D₉₀) were determined to be smaller than 54.54µm.Subsamples of Powder 1 were examined with a combination of Light Microscopy (LM) using a Nikon SWZ10A stereo microscope, SEM imaging using a FEI Quanta FEG 250 environmental SEM and by SEM-EDX analysis using a Bruker Quantax 200 Microanalysis system. Fig. 2 shows a Scanning Electron Microscopy (SEM) micrograph of Powder 1.

### Example 2 - composition of Powder 2

**Table 2**

| Component | % |
|---|---|
| Component A: Erythritol (CAS # 149-32-6) | 99.4 |
| Component B: Activated carbon (CAS# 7440-44-0) | 0.1 |
| Component C: Silica (CAS # 112945-52-5) | 0.5 |

Powder 2 is expected to be suitable for use in an air polishing device due to expected small particle size and is expected to provide a superior cleaning effect due to the presence of activated carbon.

### Example 3 - use of powder and aqueous solution in air polishing

### Enzyme aqueous solution:

**Table 3**

| Component | % |
|---|---|
| Component E: Bromelain (CAS# 37189-34-7) | 0.4 |
| Water | 99.6 |

Powder 1 was used together with the enzyme aqueous solution described in Table 3, in an EMS^{™}Airflow^{®} device, using pressure settings of 2.7 bar.

### Example 4 - use of powder and aqueous solution in an air polishing device

Powder 1 was used with 100% water in an EMS^{™}Airflow^{®} device, using pressure settings of 2.7 bar.

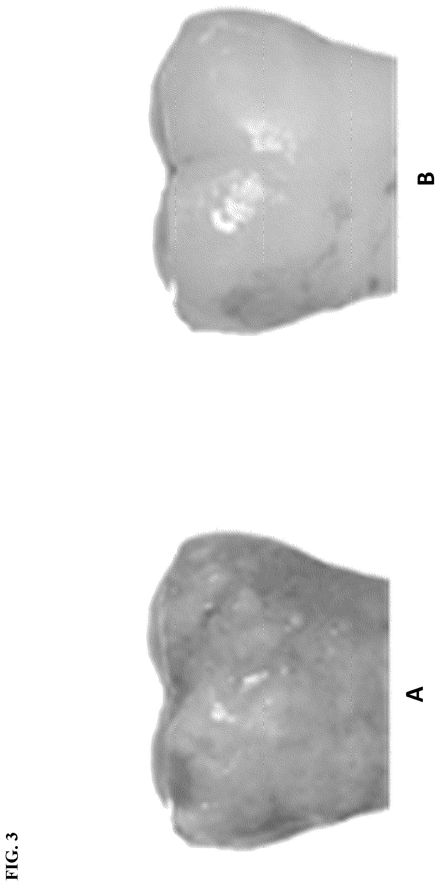

Fig. 3 shows a human molar with an artificially grown plaque (A) and a human molar after biofilm removal (B) by air polishing with Powder 1 and water. Air polishing was performed on the human molar (A) using Powder 1 in an EMS^{™}Airflow^{®} apparatus for 5 seconds, at a distance of 5 mm from the dental surface. Powder 1 and lavage with water has been shown to remove 98.9% of plaque biofilm from human molar surface (*n=8,* mean=98.94%, STDEV= 1.57).

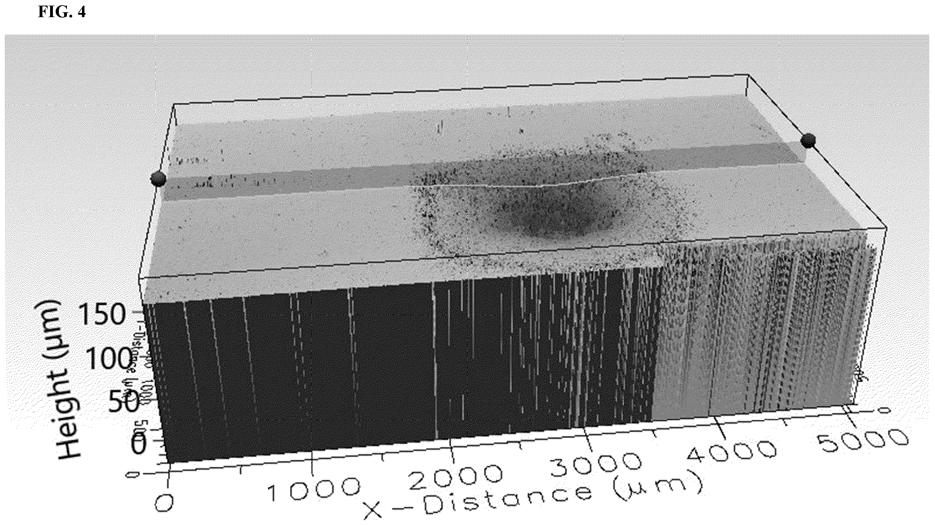

Fig. 4 shows a 3D view of post treatment dentine wear of a human molar monitored by 3D optical profilometry after application of Powder 1 and lavage with water using an EMS^{™}Airflow^{®} apparatus for 4 seconds at a distance of 5 mm from the dental surface. Mean dentine wear was found to be 3.13 µm (*n=8*, STDEV=1.68).

As demonstrated in the examples above, some demonstrative aspects include compositions or formulations which may include a powder, a liquid, and/or a combination thereof. The compositions may be used, possibly with air polishing devices, for dental care and prophylaxis. The compositions may be implemented for any other additional or alternative use, using additional or alternative suitable devices, systems, functionalities, mechanisms, and/or techniques.

Some aspects further include a method for air polishing, which includes delivering to an oral cavity an aqueous solution of one or more water-soluble enzymes via an air polishing device.

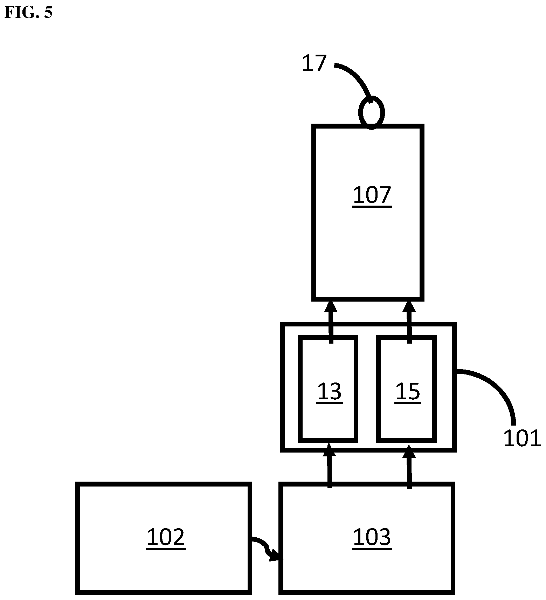

As schematically illustrated in Fig. 5, an air polishing device 100 is configured to deliver liquids and powders for the purpose of dental cleaning.

In one aspect, device 100 includes a powder compartment 13 and a liquid compartment 15, possibly, within a main body 101. Powder compartment 13 may contain a dental cleaning powder, e.g., as described herein, and liquid compartment 15 may contain water and/or an enzyme containing aqueous solution, e.g., as described herein.

In some demonstrative aspects, device 100 further includes a gas supply unit 103, typically supplying compressed air, a control unit 102, and a jetting unit 107, typically having a nozzle 17.

In some demonstrative aspects, control unit 102 may include mechanisms to control parameters of device 100 (such as pressure and temperature) and/or to enable operation of device 100, e.g., to enable delivery of gas from gas supply unit 103 to jetting unit 107. Control unit 102 may be manually operated and/or may include a processor to electronically control operation of components of device 100.

In some demonstrative aspects, device 100 is controlled, possibly via control unit 102, to jet compressed air (or another gas) from gas supply unit 103 through liquid compartment 15 carrying the aqueous solution contained in liquid compartment 15 to jetting unit 107, as schematically represented by the straight arrows. The aqueous solution is then expelled as a fine spray through nozzle 17 into the oral cavity. Other types of air polishing devices may also be implemented.

In some demonstrative aspects, the aqueous solution in liquid compartment 15 includes water soluble enzymes that can break down the components of biofilm extracellular substances, such as polysaccharides, lipids or proteins. The water-soluble enzymes may include, for example, amylase, cellulase, lysozyme, papain, bromelain and/or ficin. In some aspects, an aqueous solution may include up to about 5% w/v of one or more enzymes.

In other aspects, an aqueous solution may include up to about 2% w/v enzymes.

In some demonstrative aspects, an aqueous solution may include up to 0.4% w/v enzymes. In one example, the aqueous solution includes 0.4% w/v bromelain and 99.6% water. Other suitable enzyme concentration may be implemented.

In some demonstrative aspects, the aqueous solution includes about 0-5% w/v enzyme(s) in water. In one aspect, the aqueous solution includes about 0-0.5% w/v enzyme(s) in water. The concentration of the enzyme(s) in water may be kept below the solubility limit, e.g., to form an agglomerate-free, homogeneous solution, e.g., without sediment or gel formation. For example, the aqueous solution may be configured to flow substantially freely, and may be easily dispersed through a fluid delivery system, e.g., jetting unit 107 and nozzle 17 of air polishing device 100.

The temperature of the aqueous solution can be adjusted, e.g., by controlling a heating function of the air polishing device to meet the optimal operating temperatures of an enzyme or a blend of enzymes. For example, the operating temperature range may be from about 20°C to about 50°C. The temperature may be adjusted, for example, to meet the optimal operating temperatures of the enzymes, in accordance with the heating capacity of device 100, and/or according to a patients' preferences and tolerances.

In other aspects, any other additional or alternative design and/or mechanism may be implemented to support the flow of the aqueous solution.

Delivering enzymes via the liquid compartment of an air polishing device avoids technical challenges (e.g., large particle size, poor flowability, high abrasion) associated with delivering components through the powder compartment of the air polishing device.

As discussed above, the enzymes used according to the aspects described herein, may serve as natural biochemical compounds that can degrade components of extracellular polymeric/polysaccharide substances (EPS), which are secreted by bacteria and promote adhesion of biofilm to the dental surface. Enzymatic degradation of these components facilitates the detachment of bacterial cells. When used together with cleaning powders as described herein, cleaning may be achieved by synergistic mechanical and biochemical effects.

In some aspects, a cleaning powder (such as Powder 1 and Powder 2 described above) may be contained in powder component 13 and may be supplied, e.g., under gas pressure, to the jetting unit 107 (as schematically represented by the straight arrows) and may be then expelled through the nozzle 17 into the oral cavity. The aqueous composition and powder composition may be delivered in an independent manner of each other.

In some demonstrative aspects, the mass flow of the liquid and the powder may be controlled, for example via control unit 102, by the pressure settings of the device 100. For example, powder and liquid jetting pressures may be variable, and can be controlled, for example, by an operator and/or automatically, e.g., to meet the specific patient's treatment needs.

In some aspects, injecting the aqueous solution and expelling the powder into the oral cavity may be done substantially simultaneously. Simultaneous delivery of the aqueous solution and the powder through an orifice of the jetting unit of an air polishing device extends the arsenal of the cleaning agents beyond standard powders known in the art, to achieve a synergistic, dual-action, cleaning effect.

In other aspects, the powder and/or the aqueous solution may be delivered to the oral cavity, alone or in combination, and/or using any other additional or alternative method, procedure, and/or device. For example, the powder may be delivered to the oral cavity with the aqueous solution using other devices and in different patterns, e.g., the powder and aqueous solution may be delivered sequentially, or partially simultaneously, rather than simultaneously.

The new dual-action approach to combat biofilm, which is enabled by aspects described herein, provides, inter alia, a solution to support increased removal of antimicrobial agents which are exhibited by bacteria growing in biofilm.

### EXAMPLES

The following examples pertain to further aspects.

Example 1 includes a dental cleaning powder comprising activated carbon, wherein the activated carbon has a D₉₀ particle size of between approximately 25 µm and approximately 45µm.

Example 2 includes the subject matter of Example 1, and optionally, wherein the activated carbon has a mean particle size of 10-100 µm.

Example 3 includes the subject matter of Example 2, and optionally, wherein the activated carbon has a mean particle size of 10-30 µm.

Example 4 includes the subject matter of any one of Examples 1-3, comprising 0.1% - 5% weight of activated carbon.

Example 5 includes the subject matter of Example 4, comprising 0.1% - 0.5% weight of activated carbon.

Example 6 includes the subject matter of any one of Examples 1-5, and optionally, wherein the activated carbon comprises steam processed coconut shell.

Example 7 includes the subject matter of any one of Examples 1-6, and optionally, wherein the cleaning powder has a D₅₀ particle size of between approximately 20 µm and approximately 80 µm.

Example 8 includes the subject matter of any one of Examples 1-7, and optionally, wherein the cleaning powder has a D₉₀ particle size of approximately 54.5 µm.

Example 9 includes the subject matter of any one of Examples 1-8, and optionally, comprising an antimicrobial agent in an amount of up to approximately 3% weight.

Example 10 includes the subject matter of any one of Examples 1-9, and optionally, comprising an anticaking agent in an amount of up to approximately 10% weight.

Example 11 includes the subject matter of Example 10, and optionally, wherein the anticaking agent has a mean particle size of 1-100 µm.

Example 12 includes the subject matter of any one of Examples 1-11, and optionally, comprising a cleaning agent in an amount of between 82% to 99.5% w/w, wherein the cleaning agent comprises one or more of: a sugar alcohol, an amino acid, a sugar and an alkaline salt.

Example 13 includes the subject matter of Example 12, and optionally, wherein the cleaning agent has a mean particle size of 5-500 µm.

Example 14 includes the subject matter of any one of Examples 1-13, and optionally, comprising 99% w/w erythritol, 0.5% w/w activated carbon and 0.5% w/w silica.

Example 15 includes the subject matter of any one of Examples 1-13, and optionally, comprising 99.4% w/w erythritol, 0.1% w/w activated carbon and 0.5% w/w silica.

Example 16 includes use of the dental cleaning powder of any one of Examples 1-15 in airflow-assisted dental care.

Example 17 includes the subject matter of Example 16, and optionally, wherein the dental care is subgingival, and wherein the dental cleaning powder has a D₅₀ particle size of approximately 20 µm.

Example 18 includes the subject matter of Example 16, and optionally, wherein the dental care is supra-gingival, and wherein the dental cleaning powder has a D₅₀ particle size of approximately 80 µm.

Example 19 includes an air polishing powder comprising activated carbon particles.

Example 20 includes the subject matter of Example 19, and optionally, wherein the air polishing powder includes the dental cleaning powder of any of Examples 1-15.

Example 21 includes the subject matter of Example 19 or 20, and optionally, wherein the activated carbon particles have a mean particle size of 10-30 µm.

Example 22 includes the subject matter of Example 19 or 20, and optionally, wherein the activated carbon particles have a D₉₀ particle size of approximately 45 µm.

Example 23 includes the subject matter of any one of Examples 19-22 having a D₉₀ particle size of approximately 54.5 µm.

Example 24 includes a method for air polishing, the method comprising injecting to an oral cavity an aqueous solution of one or more water-soluble enzymes via an air polishing device.

Example 25 includes the subject matter of Example 24, and optionally, wherein the water-soluble enzymes comprise one or more of: amylase, cellulase, lysozyme, papain, bromelain and ficin.

Example 26 includes the subject matter of Example 24 or 25, and optionally, wherein the aqueous solution comprises up to 5% w/v of the one or more water-soluble enzyme.

Example 27 includes the subject matter of Example 26, and optionally, wherein the aqueous solution comprises about 0.4% w/v of the one or more water-soluble enzyme.

Example 28 includes the subject matter of any one of Examples 24-27, and optionally, wherein the aqueous solution comprises 0.4% w/v bromelain and 99.6% water.

Example 29 includes the subject matter of any one of Examples 24-28, and optionally, comprising expelling to the oral cavity, via the air polishing device, a powder comprising activated carbon particles.

Example 30 includes the subject matter of Example 29, and optionally, comprising injecting the aqueous solution and expelling the powder substantially simultaneously.

Example 31 includes the subject matter of Example 29 or 30, and optionally, wherein the powder includes the dental cleaning powder of any of Examples 1-15.

Functions, operations, components and/or features described herein with reference to one or more aspects, may be combined with, or may be utilized in combination with, one or more other functions, operations, components and/or features described herein with reference to one or more other aspects, or vice versa.

While certain features have been illustrated and described herein, many modifications, substitutions, changes, and equivalents may occur to those skilled in the art. It is, therefore, to be understood that the appended claims are intended to cover all such modifications and changes as fall within the true spirit of the disclosure.

## Claims

1. A dental cleaning powder comprising activated carbon, wherein the activated carbon has a D₉₀ particle size of between approximately 25 µm and approximately 45µm.

2. The dental cleaning powder of claim 1, wherein the activated carbon has a mean particle size of 10-100 µm.

3. The dental cleaning powder of claim 2, wherein the activated carbon has a mean particle size of 10-30 µm.

4. The dental cleaning powder of any one of claims 1-3, comprising 0.1% - 5% weight of activated carbon.

5. The dental cleaning powder of claim 4, comprising 0.1% - 0.5% weight of activated carbon.

6. The dental cleaning powder of any one of claims 1-5, wherein the activated carbon comprises steam processed coconut shell.

7. The dental cleaning powder of any one of claims 1-6, wherein the cleaning powder has a D₅₀ particle size of between approximately 20 µm and approximately 80 µm, and/or wherein the cleaning powder has a D₉₀ particle size of approximately 54.5 µm.

8. The dental cleaning powder of any one of claims 1-7 comprising an antimicrobial agent in an amount of up to approximately 3% weight, and/or an anticaking agent in an amount of up to approximately 10% weight.

9. The dental cleaning powder of any one of claims 1-8 comprising a cleaning agent in an amount of between 82% to 99.5% w/w, wherein the cleaning agent comprises one or more of: a sugar alcohol, an amino acid, a sugar and an alkaline salt.

10. The dental cleaning powder of any one of claims 1-9 comprising 99% w/w erythritol, 0.5% w/w activated carbon and 0.5% w/w silica, or comprising 99.4% w/w erythritol, 0.1% w/w activated carbon and 0.5% w/w silica.

11. Use of the dental cleaning powder of any one of claims 1-10 in airflow-assisted dental care.

12. The use of claim 11, wherein the dental care is subgingival, and wherein the dental cleaning powder has a D₅₀ particle size of approximately 20 µm, or wherein the dental care is supra-gingival, and wherein the dental cleaning powder has a D₅₀ particle size of approximately 80 µm.

13. An air polishing powder comprising activated carbon particles, wherein the activated carbon particles have a mean particle size of 10-30 µm, and/or wherein the activated carbon particles have a D₉₀ particle size of approximately 45 µm, and/or wherein the air polishing powder has a D₉₀ particle size of approximately 54.5 µm.

14. A method for air polishing, the method comprising injecting to an oral cavity an aqueous solution of one or more water-soluble enzymes via an air polishing device, and expelling to the oral cavity, via the air polishing device, a powder comprising activated carbon particles.

15. The method of claim 14, wherein the water-soluble enzymes comprise one or more of: amylase, cellulase, lysozyme, papain, bromelain and ficin, and/or wherein the aqueous solution comprises up to 5% w/v of the one or more water-soluble enzyme, and/or wherein the aqueous solution comprises 0.4% w/v bromelain and 99.6% water, and/or wherein the method comprises injecting the aqueous solution and expelling the powder substantially simultaneously.
